# EUROPEAN PATENT APPLICATION

(11) **EP 2 361 655 A1**
(43) Date of publication of application: **31.08.2011**
(21) Application number: 11155959.7
(22) Date of filing: 25.02.2011
(51) Int. Cl.: A61N 1/40, A61B 18/12, A61N 5/04

(54) **Patient isolation in a microwave - radio frequency generator**

(30) Priority: 25.02.2010 US 712712
(71) Applicant: Tyco Healthcare Group, LP, Boulder, CO 80301 (US)
(72) Inventor: Prakash, Mani N., Boulder, CO 80301 (US); Behnke, Robert J., Erie, CO 80516 (US)
(74) Representative: HOFFMANN EITLE

(57) **Abstract**

The present disclosure provides an ablation system. The ablation system includes a generator having a first energy source that supplies a first type of energy to tissue. The generator also has a second energy source that supplies a second type of energy to tissue different from the first type of energy. A diplexer is also provided that is operable to multiplex the first type of energy from the first energy source and the second type of energy from the second energy source and provide an output to an ablation device. Additionally, the generator includes a first isolation device coupled to the first energy source and the diplexer, and a second isolation device coupled to the second energy source and the diplexer.

## Description

### BACKGROUND

### 1. Technical Field

The present disclosure relates generally to microwave ablation procedures that utilize microwave surgical devices having a microwave antenna that may be inserted directly into tissue for diagnosis and treatment of diseases. More particularly, the present disclosure is directed to a multi-purpose generator that utilizes microwave and radio frequency energy.

### 2. Background of Related Art

In the treatment of diseases such as cancer, certain types of cancer cells have been found to denature at elevated temperatures (which are slightly lower than temperatures normally injurious to healthy cells.) These types of treatments, known generally as hyperthermia therapy, typically utilize electromagnetic radiation to heat diseased cells to temperatures above 41 °C, while maintaining adjacent healthy cells at lower temperatures where irreversible cell destruction will not occur. Other procedures utilizing electromagnetic radiation to heat tissue also include ablation and coagulation of the tissue. Such microwave ablation procedures, e.g., such as those performed for menorrhagia, are typically done to ablate and coagulate the targeted tissue to denature or kill the tissue. Many procedures and types of devices utilizing electromagnetic radiation therapy are known in the art. Such microwave therapy is typically used in the treatment of tissue and organs such as the prostate, heart, liver, lung, kidney, and breast.

One non-invasive procedure generally involves the treatment of tissue (e.g., a tumor) underlying the skin via the use of microwave energy. The microwave energy is able to non-invasively penetrate the skin to reach the underlying tissue. However, this non-invasive procedure may result in the unwanted heating of healthy tissue. Thus, the non-invasive use of microwave energy requires a great deal of control.

Presently, there are several types of microwave probes in use, e.g., monopole, dipole, and helical. One type is a monopole antenna probe, which consists of a single, elongated microwave conductor exposed at the end of the probe. The probe is typically surrounded by a dielectric sleeve. The second type of microwave probe commonly used is a dipole antenna, which consists of a coaxial construction having an inner conductor and an outer conductor with a dielectric junction separating a portion of the inner conductor. The inner conductor may be coupled to a portion corresponding to a first dipole radiating portion, and a portion of the outer conductor may be coupled to a second dipole radiating portion. The dipole radiating portions may be configured such that one radiating portion is located proximally of the dielectric junction, and the other portion is located distally of the dielectric junction. In the monopole and dipole antenna probe, microwave energy generally radiates perpendicularly from the axis of the conductor.

The typical microwave antenna has a long, thin inner conductor that extends along the axis of the probe and is surrounded by a dielectric material and is further surrounded by an outer conductor around the dielectric material such that the outer conductor also extends along the axis of the probe. In another variation of the probe that provides for effective outward radiation of energy or heating, a portion or portions of the outer conductor can be selectively removed. This type of construction is typically referred to as a "leaky waveguide" or "leaky coaxial" antenna. Another variation on the microwave probe involves having the tip formed in a uniform spiral pattern, such as a helix, to provide the necessary configuration for effective radiation. This variation can be used to direct energy in a particular direction, e,g., perpendicular to the axis, in a forward direction (i.e., towards the distal end of the antenna), or combinations thereof.

Invasive procedures and devices have been developed in which a microwave antenna probe may be either inserted directly into a point of treatment via a normal body orifice or percutaneously inserted. Such invasive procedures and devices potentially provide better temperature control of the tissue being treated. Because of the small difference between the temperature required for denaturing malignant cells and the temperature injurious to healthy cells, a known heating pattern and predictable temperature control is important so that heating is confined to the tissue to be treated. For instance, hyperthermia treatment at the threshold temperature of about 41.5°C generally has little effect on most malignant growth of cells. However, at slightly elevated temperatures above the approximate range of 43°C to 45°C, thermal damage to most types of normal cells is routinely observed. Accordingly, great care must be taken not to exceed these temperatures in healthy tissue.

In the case of tissue ablation, a microwave frequency electrical current in the range of about 500 MHz to about 10 GHz is applied to a targeted tissue site to create an ablation volume, which may have a particular size and shape. Ablation volume is correlated to antenna design, antenna performance, antenna impedance and tissue impedance. The particular type of tissue ablation procedure may dictate a particular ablation volume in order to achieve a desired surgical outcome. By way of example, and without limitation, a spinal ablation procedure may call for a longer, narrower ablation volume, whereas in a prostate ablation procedure, a more spherical ablation volume may be required.

One common design practice used to ensure patient safety in electrosurgical generators is to create an isolation barrier between the generator and the patient. This is accomplished by isolating the generator output from an earth ground. isolation barriers may be created by various generally accepted circuits, such as, for example, a transformer or capacitors that would have a low impedance at about 60Hz.

Microwave ablation devices may utilize a multi-function generator that uses two different frequencies during a surgical procedure. The generator may use radio frequency (RF) for cutting and coagulation and microwave frequencies for ablation. When a multi-function generator is used, isolation of the different frequencies to the patient can become difficult. This is due in part to the difference between the operating frequencies for RF and microwave. The operating frequencies are so far apart between RF and microwave that the isolation barrier that works for one frequency will not work for the other frequency.

### SUMMARY

The present disclosure provides a microwave ablation system. In embodiments of the present disclosure, the microwave ablation system includes a generator having a first energy source that supplies a first type of energy to tissue. The generator also has a second energy source that supplies a second type of energy to tissue. A diplexer is also provided that is operable to multiplex the first type of energy from the first energy source and the second type of energy from the second energy source and provide an output to an ablation device. Additionally, the generator includes a first isolation device, which is coupled to the first energy source and the diplexer, and a second isolation device, which is coupled to the second energy source and the diplexer.

The system may also have a controller operable to control the first energy source and the second energy source. The first energy source is a microwave generator and the second energy source is a radio frequency generator. The first and second isolation devices may transfer energy by inductive coupling, capacitive coupling or antenna to antenna energy transfer.

The present disclosure also provides another microwave ablation system. The microwave ablation system includes a generator including a first energy source that supplies a first type of energy to tissue, a second energy source that supplies a second type of energy to tissue, a power source that powers the first and second energy source and a user interface. The system also includes a cable having an inner conductor that transmits the first type of energy to tissue, an outer conductor that transmits a second type of energy to tissue and a dielectric material disposed therebetween. At least one capacitor is placed in series with a first portion of the inner conductor and a second portion of the inner conductor, and at least one capacitor is placed in series with a first portion of the outer conductor and a second portion of the outer conductor.

In another embodiment, the microwave ablation system may also have a shielding material around the cable.

In another embodiment, the user interface may include a controller operable to control the first energy source and the second energy source. The first energy source is a microwave generator and the second energy source is a radio frequency generator.

In another embodiment, each capacitor is a discrete component or is formed from a layer of dielectric material.

### BRIEF DESCRIPTION OF THE DRAWINGS

The above and other aspects, features, and advantages of the present disclosure will become more apparent in light of the following detailed description when taken in conjunction with the accompanying drawings in which:

Fig. 1 shows a representative diagram of a variation of a microwave antenna assembly in accordance with an embodiment of the present disclosure;

Fig. 2 shows a representative diagram of a generator for use in a microwave ablation system in accordance with an embodiment of the present disclosure;

Fig. 3 shows a representative diagram of a microwave ablation system in accordance with an embodiment of the present disclosure;

Figs. 4A and 4B show representative schematic diagrams of a coaxial cable for use in a microwave ablation system in accordance with an embodiment of the present disclosure;

Fig. 5 shows a representative diagram of a variation of a coaxial cable for use in a microwave ablation system in accordance with an embodiment of the present disclosure;

Fig. 6 shows a representative diagram of a variation of a coaxial cable for use in a microwave ablation system in accordance with an embodiment of the present disclosure; and

Fig. 7 is a schematic diagram of a microwave ablation system in accordance with an embodiment of the present disclosure.

### DETAILED DESCRIPTION

Particular embodiments of the present disclosure are described hereinbelow with reference to the accompanying drawings; however, it is to be understood that the disclosed embodiments are merely exemplary of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure. Like reference numerals may refer to similar or identical elements throughout the description of the figures. As shown in the drawings and described throughout the following description, as is traditional when referring to relative positioning on a surgical instrument, the term "proximal" refers to the end of the apparatus which is closer to the user and the term "distal" refers to the end of the apparatus which is further away from the user.

Electromagnetic energy is generally classified by increasing energy or decreasing wavelength into radio waves, microwaves, infrared, visible light, ultraviolet, X-rays and gamma-rays. As used herein, the term "microwave" generally refers to electromagnetic waves in the frequency range of 300 megahertz (MHz) (3 x 10⁸ cycles/second) to 300 gigahertz (GHz) (3 x 10¹¹ cycles/second). As used herein, the term "RF'' generally refers to electromagnetic waves having a lower frequency than microwaves. The phrase "ablation procedure" generally refers to any ablation procedure, such as microwave ablation or microwave ablation assisted resection. The phrase "transmission line" generally refers to any transmission medium that can be used for the propagation of signals from one point to another.

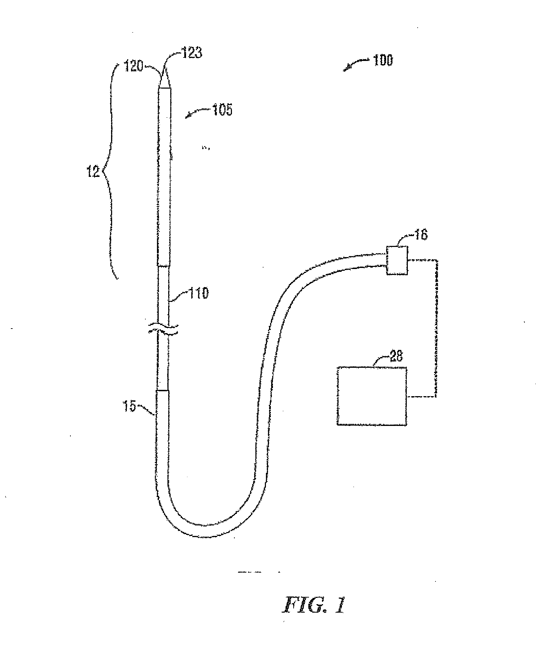

Fig. 1 shows an embodiment of a microwave antenna assembly 100 in accordance with one embodiment of the present disclosure. Antenna assembly 100 includes a radiating portion 12 that is connected by feedline 110 (or shaft) via cable 15 to connector 16, which may further connect the assembly 100 to a power generating source 28, e.g., a microwave and/or RF electrosurgical generator. Assembly 100, as shown, is a dipole microwave antenna assembly, but other antenna assemblies, e.g., monopole or leaky wave antenna assemblies, may also utilize the principles set forth herein. Distal radiating portion 105 of radiating portion 12 includes a tapered end 120 which terminates at a tip 123 to allow for insertion into tissue with minimal resistance. It is to be understood, however, that tapered end 120 may include other shapes, such as without limitation, a tip 123 that is rounded, flat, square, hexagonal, or cylindroconical.

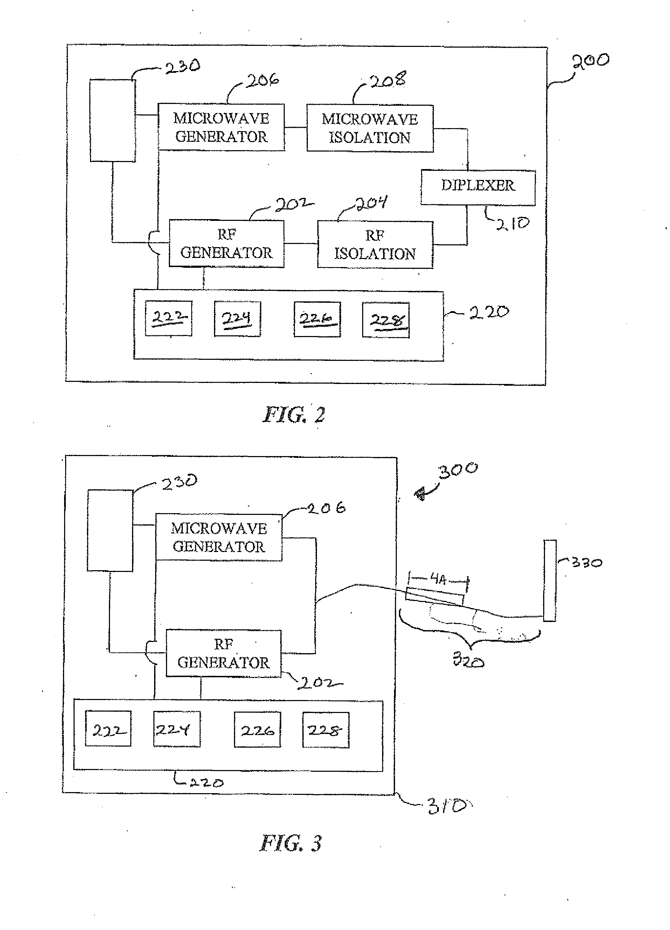

Fig. 2 shows a microwave/RF generator 200 in accordance with an embodiment of the present disclosure. Generator 200 includes an RF generator 202 that outputs RF energy and is coupled to an RF isolation device 204. Generator 200 also includes a microwave generator 206 that outputs microwave energy and is coupled to a microwave isolation device 208. Isolation devices 204 and 208 may be any suitable device that transfers energy from a first electrical circuit (energy source) to a second electrical circuit (e.g., an electrical load) without direct electrical contact, such as, for example, by inductive coupling, capacitive coupling or antenna to antenna energy transfer (wireless).

RF isolation device 204 and microwave isolation device 208 are coupled to a diplexer 210 that combines the RF energy with the microwave energy. Diplexer 210 implement frequency domain multiplexing where two ports are multiplexed onto a third port. The diplexer 210 blocks the RF energy from getting into the microwave energy source 206 and blocks microwave energy from getting into the RF energy source 202. Diplexer 210 allows both the RF energy and the microwave energy to flow to antenna assembly 100 simultaneously.

A user interface 220 is provided to control the output of the generator 200 and allows a user to select the output of the generator 200. A user may select to output microwave energy, RF energy or both microwave energy and RF energy simultaneously. User interface 220 may have an input device 222 such as a keyboard, switches, buttons, a liquid crystal display (LCD) touch panel used by a user to control the generator 200. User interface 220 may also include a memory 224 such as random access memory (RAM) or read only memory (ROM) that stores a program used to control the generator. User interface may also include a processor 226 used to receive inputs from a user and generate a signal used to control the generator based on the user's inputs. The user interface 220 may also have a display 228 configured to display a status of the generator 200. The display 228 may be a LCD, a light emitting diode (LED) display or any number of indicator lights.

Generator 200 also includes a power source 230 that provides power to RF generator 202 and microwave generator 206. Power source 230 may be a battery or a power supply that is connected to an AC line.

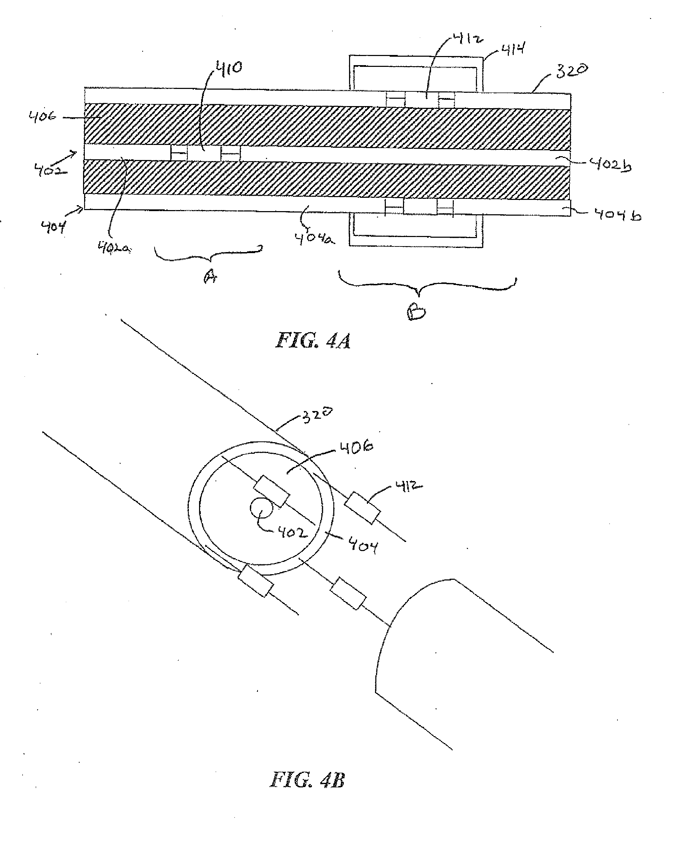

Fig. 3 shows a microwave ablation system 300 in accordance with another embodiment of the present disclosure. In system 300, the patient isolation is provided in the cable rather than in the generator as shown in Fig. 2. System 300 includes a generator 310 which also includes an RF generator 202, a microwave generator 206, a user interface 220 and a power source 230 as described above. Generator 310 is coupled to a probe 330 via a coaxial cable 320. Coaxial cable 320 provides the patient isolation as will be described below with regard to Figs. 4A and 4B.

Fig. 4A shows a schematic cross section of the coaxial cable 320 of Fig. 3. As shown in Fig. 4A, coaxial cable 320 includes an inner conductor 402, an outer conductor 404 and a dielectric material 406. Patient isolation provided for the inner conductor is shown in region "A" of coaxial cable 320. In region "A", capacitor 410 is placed in series between inner conductor portion 402a and inner conductor portion 402b. Capacitor 410 provides the isolation between the generator and the patient. Patient isolation provided for the outer conductor is shown in region "B". In region "B", any number of capacitors 412 are placed in series between outer conductor portion 404a and outer conductor portion 404b. Capacitors 410 and 412 can be any discrete components or distributed capacitors formed by an appropriate layer of dielectric material. Additional shielding 414 may be provided to minimize unwanted radiation. Shielding 414 may be formed from most any conductive material such as a metal or metal alloy, including but not limited to, gold, copper, aluminum or stainless steel.

Fig. 4B shows another cross section of the coaxial cable in region B of coaxial cable 320. As shown in Fig. 4B, capacitors 412 are distributed on all sides of outer conductor 404. The capacitors are chosen such that they form a short circuit at the higher frequencies being used and result in an open circuit at the lower frequencies.

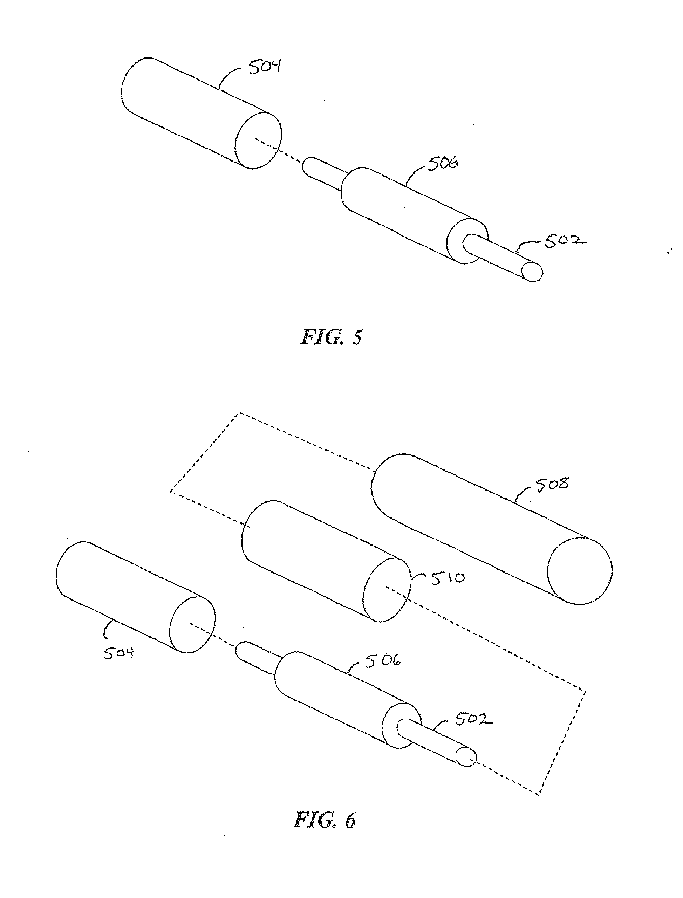

Figs. 5 and 6 show an example of another isolation device that can be used with a coaxial cable in accordance with an embodiment of the present disclosure. Fig. 5 shows a center rod 502 that is fed into a center conductor 504. A dielectric boundary 506 is formed in the center conductor by insulating rod 502 with multiple layers of high voltage plastic. The insulated rod 502 and the center conductor 504 form a first capacitance coupling C1. The coupling is dependent on the amount of insulating rod 502 overlapped by center conductor 504. As shown in Fig. 6, an outer conductor 508 is provided that has a larger diameter than center conductor 504. When assembled, outer conductor 508 is separated from center conductor 504 by a white plastic material 510 that acts as an outer dielectric. Outer conductor 508 and center conductor 504 form a second capacitance coupling C2. Insulated tape (not shown) may be used on outer conductor 508 and center conductor 504 to ensure that the two conductors are electrically isolated.

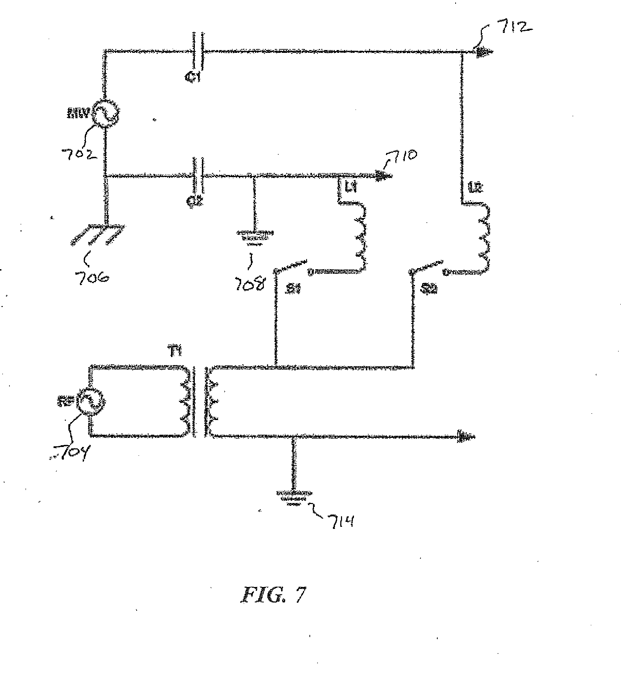

Fig. 7 shows a schematic diagram of a microwave ablation system that utilizes the isolation device of Figs. 5 and 6 in accordance with an embodiment of the present disclosure. As shown in Fig. 7, a microwave generator 702 and an RF generator 704 is provided. C1 represents the capacitance coupling between the insulated rod 502 and the center conductor 504 of Figs. 5 and 6. C2 represents the capacitance coupling between outer conductor 508 and center conductor 504. Ground 706 represents the earth potential while ground 708 represents the patient ground. Capacitance coupling C2 provides the isolation between the microwave generator and the patient.

Transformer T1 provides RF isolation that meets desired electrical parameters and safety between RF generator 704 and a patient. The RF energy can be fed into the microwave energy either on the outer conductor 710 or the center conductor 712. If switch S I is closed, then the RF energy is fed through inductor L1 into the outer conductor 710. If switch S2 is closed, then the RF energy is fed through inductor L2 into the center conductor 712. Inductors L1 and L2 block microwave energy from entering into RF generator 704. RF patient ground 714 can be coupled to a return pad (not shown) or could be directed to the antenna assembly (not shown).

The schematic diagram of Fig. 7 provides a microwave isolation technique where the barrier will be large enough to pass any safety regulations regarding creepage and clearance. Further, the voltage breakdown will be high enough to meet the high voltage demands.

The described embodiments of the present disclosure are intended to be illustrative rather than restrictive, and are not intended to represent every embodiment of the present disclosure. Various modifications and variations can be made without departing from the spirit or scope of the disclosure as set forth in the following claims both literally and in equivalents recognized in law.

## Claims

1. An ablation system comprising:
a first energy source that supplies a first type of energy;
a second energy source that supplies a second type of energy different from the first type of energy;
a diplexer configured to multiplex the first type of energy from the first energy source and the second type of energy from the second energy source and provide an output to an ablation device;
a first isolation device operably coupled to the first energy source and the diplexer; and
a second isolation device operably coupled to the second energy source and the diplexer.

2. The ablation system according to claim 1 further comprising a controller configured to control the first energy source and the second energy source.

3. The ablation system according to claim 1 wherein the first energy source is a microwave generator.

4. The ablation system according to claim 1, wherein at least one of the isolation devices transfers energy by inductive coupling, capacitive coupling or antenna to antenna energy transfer.

5. The ablation system according to claim 1 wherein the second energy source is a radio frequency generator.

6. A microwave ablation system comprising:
a generator including a first energy source that supplies a first type of energy, a second energy source that supplies a second type of energy different from the first type of energy, and a power source that powers the first and second energy sources and a user interface;
a cable including an inner conductor configured to transmit the first type of energy, an outer conductor configured to transmit the second type of energy and a dielectric material disposed therebetween;
at least one capacitor in series with a first portion of the inner conductor and a second portion of the inner conductor; and
at least one capacitor in series with a first portion of the outer conductor and a second portion of the outer conductor.

7. The microwave ablation system according to claim 6 further comprising a shielding material around the cable.

8. The microwave ablation system according to claim 6 wherein the user interface includes a controller configured to control the first energy source and the second energy source.

9. The microwave ablation system according to claim 6 wherein each capacitor is a discrete component.

10. The microwave ablation system according to claim 6 wherein each capacitor is formed from a layer of dielectric material.

11. The microwave ablation system according to claim 6 wherein the first energy source is a microwave generator.

12. The microwave ablation system according to claim 6 wherein the second energy source is a radio frequency generator.
